# EUROPÄISCHE PATENTSCHRIFT

(11) **EP 3 111 919 B1**
(45) Veröffentlichungstag und Bekanntmachung des Hinweises auf die Patenterteilung: **21.03.2018**
(21) Anmeldenummer: 15174954.6
(22) Anmeldetag: 02.07.2015
(51) Int. Cl.: A61K 8/41, A61Q 19/00, A61K 8/06

(54) **EMULSIONEN ENTHALTEND KATIONISCHE EMULGATOREN AUF BASIS VON MDIPA ESTERQUATS**
EMULSIONS CONTAINING CATIONIC EMULSIFIERS BASED ON MDIPA ESTERQUATS
ÉMULSIONS CONTENANT DES ÉMULSIFIANTS CATIONIQUES À BASE D'ESTERQUATS MDIPA

(43) Veröffentlichungstag der Anmeldung: 04.01.2017
(73) Patentinhaber: Evonik Degussa GmbH, 45128 Essen (DE)
(72) Erfinder: MEYER, Juergen, 45134 Essen (DE); HOWE, Anna M., 23120 Moseley, VA (US); SPOHRER, Maria L, 23059 Glen Allen, VA (US); KÖHLE, Hans-Jürgen, 63533 Mainhausen (DE); JHA, Brajesh Kumar, 23112 Midlothian, VA (US)

(56) Entgegenhaltungen:
- EP-A2- 1 844 753
- EP-A2- 2 783 677
- WO-A1-2004/093834

## Beschreibung

### Gebiet der Erfindung

Gegenstand der Erfindung sind Emulsionen enthaltend bestimmte Alkylesterquats, Konsistenzgeber und kosmetisches Öl.

### Stand der Technik

Die Erfindung ermöglicht die Herstellung langzeitstabiler kosmetischer Emulsionen mit einem angenehm trockenen Hautgefühl bei gleichzeitig deutlich verbesserter Bioabbaubarkeit und Ecotoxizität des eingesetzten kationischen Emulgators im Vergleich zum Stand der Technik.

Kationische Emulsionen werden seit vielen Jahren vor allem in Nordamerika, zunehmend aber auch in anderen Regionen, insbesondere im Bereich der kosmetischen Hautpflege, eingesetzt. Der besondere Vorteil dieser Systeme liegt darin, dass sie selbst bei Einsatz extrem reichhaltiger Öle wie Petrolatum oder Mineralöl eine schnelle Absorption in die Haut zeigen und ein sehr trockenes Hautgefühl hinterlassen. Weiterhin zeichnen sich diese Emulsionen dadurch aus, dass sie eine langanhaltende feuchtigkeitsspendende Wirkung aufweisen. Darüber hinaus zeigen sie filmbildende Eigenschaften, die sie prinzipiell auch für Sonnenschutzanwendungen interessant machen.

US4389418 beschreibt Hautpflegezusammensetzungen auf Basis von Petrolatum oder Mineralöl, eines kationischen Emulgators, Fettalkohols und eines Esteröls. Diese Zusammensetzungen haben feuchtigkeitsspendende Eigenschaften, zeigen aber gleichzeitig auch eine nicht-fettige, angenehme Sensorik bei Applikation auf der Haut. Die in US4389418 beschriebenen Emulsionen basieren auf Dimethyldialkylammoniumsalzen, bevorzugt auf Dimethyldistearylammoniumchloride (Distearyldimonium Chloride).
Aufgrund der sehr guten Emulgierleistung dieses Emulgators und der entsprechenden Lagerstabilität und Flexibilität dieser Emulsionen ist Distearyldimonium Chloride bis heute der hauptsächlich verwendete Emulgator für kationische Emulsionen für kosmetische Anwendungen.

In den letzten Jahren bekommen allerdings Fragen der Nachhaltigkeit und Umweltverträglichkeit kosmetischer Produkte zunehmende Bedeutung. Kosmetische Inhaltsstoffe sollen sich vorzugsweise durch eine gute Bioabbaubarkeit und geringe Umwelttoxizität auszeichnen. Selbstverständlich sollen diese Eigenschaften möglicher kationischer Emulgatoren verbessert, die Anwendungseigenschaften der damit hergestellten kosmetischen Emulsionen aber in keiner Weise eingeschränkt werden.

Bisher beschriebene mögliche Alternativen für solche kationischen Emulsionssysteme für kosmetische Anwendungen sind vor allem Esterquats, die durch Veresterung von Fettsäuren mit einer Kettenlänge von C16 - C22 mit Triethanolamin oder Methyldiethanolamin und nachfolgender Quaternierung erhältlich sind.
US7597881 beschreibt Sonnenschutzemulsionen vor allem auf Basis solcher Esterquats, die eine sandabweisende Wirkung aufweisen.

Allerdings zeigen Emulsionen auf Basis solcher Esterquats Stabilitätsprobleme bei Langzeitlagerung insbesondere bei höheren Temperaturen. Aus diesem Grund hat sich die Verwendung von Esterquats in kosmetischen Emulsionen für Applikationen auf der Haut bis heute nicht am Markt etabliert.

EP2783677 beschreibt kosmetische Formulierungen enthaltend Esterquats auf Isopropanolaminbasis, sowie die Verwendung dieser Esterquats in der Kosmetik. Die beschriebenen Esterquats enthalten dabei Alkylreste mit mindestens einer ungesättigten Fettsäure mit einer Kettenlänge von 18 bis 24 C-Atomen oder einen Acylrest der Isostearinsäure oder Rizinolsäure. Solche Esterquats eignen sich für kosmetische Haarpflegeprodukte. Allerdings zeigen sie keine ausreichende Stabilisierung von kosmetischen Emulsionen und sind somit zur Herstellung kationischer Emulsionen für Hautpflegeanwendungen nicht geeignet.

Aufgabe der Erfindung war es somit, langzeitstabile Emulsionen auf Basis kationischer Emulgatoren zu entwickeln, die eine deutlich bessere Emulsionsstabilisierung ermöglichen, als die bisher beschriebenen Systeme auf Esterquatbasis.

### Beschreibung der Erfindung

Überraschenderweise wurde gefunden, dass die im Folgenden beschriebenen Emulsionen die der Erfindung gestellte Aufgabe zu lösen vermögen.

Gegenstand der vorliegenden Erfindung sind daher Emulsionen wie in Anspruch 1 beschrieben.
Ein weiterer Gegenstand der Erfindung ist die Verwendung von speziellen Alkylesterquats als Emulgatoren.

Ein Vorteil der vorliegenden Erfindung ist, dass die in den erfindungsgemäßen Emulsionen enthaltenen Alkylesterquats ein deutlich besseres Umweltprofil aufweisen als Distearyldimonium Chloride, dem bisherigen Goldstandard der kationischen Emulgatoren im Kosmetikbereich.
Ein Vorteil der Erfindung ist, dass die erfindungsgemäßen Emulsionen ein vorteilhaftes angenehm leichtes, nicht klebriges Hautgefühl zeigen. Dieses Hautgefühl entspricht vorteilhafterweise dabei dem Hautgefühl, das typischerweise auch Systeme mit Distearyldimonium Chloride aufweisen. Somit können klassische Emulsionssysteme auf Basis von Distearyldimonium Chloride in einfacher Art und Weise in umweltfreundlichere Emulsionssysteme mit vergleichbaren Anwendungseigenschaften überführt werden.
Ein weiterer Vorteil der vorliegenden Erfindung ist, dass die in den erfindungsgemäßen Emulsionen eingesetzten Alkylesterquats in der Regel komplett frei von Lösungsmitteln hergestellt werden können, während das typischerweise in der Kosmetik eingesetzte Distearyldimonium Chloride kleine Restmengen von Lösungsmitteln wie z.B. Ispropylalkohol enthält.
Vorteilhaft ist insbesondere auch die große Formulierungsflexibilität der erfindungsgemäßen Emulsionen, so dass sich leicht maßgeschneiderte Formulierungen für ein breites Feld von Anwendungen auf Basis dieser Emulsionen entwickeln lassen. Dabei können im Bereich Sonnenschutz und Make-up insbesondere Vorteile hinsichtlich der Wasserfestigkeit dieser Formulierungen gegenüber anderen Formulierungskonzepten beobachtet werden.

Gegenstand der vorliegenden Erfindung ist somit eine Emulsion enthaltend
A) Alkylesterquats der allgemeinen Formel I 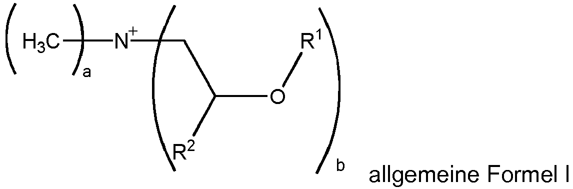
   mit R¹ ein Acylrest einer Fettsäure mit einer Kettenlänge von 6 bis 24 Kohlenstoffatomen, bevorzugt 12 bis 22 Kohlenstoffatomen, insbesondere 16 bis 18 Kohlenstoffatomen, mit der Maßgabe, dass der Massenanteil von gesättigten, linearen Fettsäuren mit einer Kettenlänge von 12 bis 24 Kohlenstoffatomen, bevorzugt 14 bis 22 Kohlenstoffatomen, insbesondere 16 bis 18 Kohlenstoffatomen, mehr als 50 Gew.-%, bevorzugt mehr als 55 Gew.-%, ganz besonders bevorzugt mehr als 60 Gew.-%, bezogen auf alle Acylreste R¹ beträgt,
   mit R² ein Alkylrest mit 1 bis 6 Kohlenstoffatomen, bevorzugt Methyl, Ethyl, Propyl, Isopropyl, besonders bevorzugt Methyl,
   wobei a = 1-3 und b = 1-3, bevorzugt a = 1,8-2,2 und b = 1,8-2,2, besonders bevorzugt a = b = 2, mit der Maßgabe, dass a + b = 4,
B) mindestens einen Konsistenzgeber, und
C) mindestens ein kosmetisches Öl.

Umfasst sind offensichtlich Mischungen von Esterquats, die in den erfindungsgemäßen Emulsionen enthalten sein können; Angaben zu Anzahl von Einheiten (speziell die Werte a und b), die in den Esterquats mehrfach enthalten sein können, sind als Mittelwert, gemittelt über alle entsprechenden Verbindungen zu verstehen.
Die Emulsionen enthalten z.B. die Komponenten A), B) und C). Bevorzugte Ausführungsformen der erfindungsgemäßen Emulsionen, bei denen nur noch Teilbereiche der Komponenten A), B) oder C) enthalten sind, sind natürlich zwangsläufig frei von Komponenten der weniger bevorzugten Bereiche.
So enthalten z.B. bevorzugte Emulsionen, bei denen A) Alkylesterquats der allgemeinen Formel I mit R¹ ein Acylrest einer Fettsäure mit einer Kettenlänge von 12 bis 22 Kohlenstoffatomen enthalten sind, keine Alkylesterquats der allgemeinen Formel I mit R¹ Acylrest einer Fettsäure mit einer Kettenlänge von 6 bis 11 und 23 bis 24 Kohlenstoffatomen.

Alle angegebenen Prozent (%) sind, wenn nicht anders angegeben, Massenprozent.

Es ist erfindungsgemäß bevorzugt, wenn der Massenanteil an Resten R¹ von ungesättigten, linearen Fettsäuren mit einer Kettenlänge von 12 bis 24 Kohlenstoffatomen, bevorzugt 14 bis 22 Kohlenstoffatomen, insbesondere 16 bis 18 Kohlenstoffatomen, von 5 Gew.-% bis 35 Gew.-%, bevorzugt von 10 Gew.-% bis 30 Gew.-%, ganz besonders bevorzugt von 15 Gew.-% bis 25 Gew.-%, bezogen auf alle Acylreste R¹ beträgt.

Bevorzugte erfindungsgemäße Emulsionen sind dadurch gekennzeichnet, dass
A) in einer Menge von 0,2 Gew.-% bis 10 Gew.-%, bevorzugt von 0,5 Gew.-% bis 7 Gew.-% insbesondere von 1 Gew.-% bis 6 Gew.-%,
B) in einer Menge von 0,5 Gew.-% bis 10 Gew.-%, bevorzugt von 2 Gew.-% bis 8 Gew.-% insbesondere von 3 Gew.-% bis 6 Gew.-%, und
C) in einer Menge von 5 Gew.-% bis 40 Gew.-%, bevorzugt von 6 Gew.-% bis 35 Gew.-% insbesondere von 8 Gew.-% bis 30 Gew.-%,
wobei sich die Gewichtsprozent auf die Gesamtemulsion beziehen.

Besonders bevorzugte erfindungsgemäße Emulsionen enthalten
A) Alkylesterquats der allgemeinen Formel I
   mit R¹ ein Acylrest einer Fettsäure mit einer Kettenlänge von 6 bis 24, mit der Maßgabe, dass der Massenanteil von gesättigten, linearen Fettsäuren mit einer Kettenlänge von 16 bis 18 Kohlenstoffatomen, mehr als 60 Gew.-%, bezogen auf alle Acylreste R¹ beträgt,
   mit R² Methyl,
   wobei a = 1,8-2,2 und b = 1,8-2,2, mit der Maßgabe, dass a + b = 4.
In diesem Zusammenhang ist es insbesondere bevorzugt, wenn der Massenanteil an Resten R¹ von ungesättigten, linearen Fettsäuren von 16 bis 18 Kohlenstoffatomen von 15 Gew.-% bis 30 Gew.-% bezogen auf alle Acylreste R¹ beträgt.

Ganz besonders bevorzugte erfindungsgemäße Emulsionen enthalten
A) Alkylesterquats der allgemeinen Formel I
   mit R¹ ein Acylrest einer Fettsäure mit einer Kettenlänge von 6 bis 24, mit der Maßgabe, dass der Massenanteil von gesättigten, linearen Fettsäuren mit einer Kettenlänge von 18 Kohlenstoffatomen, mehr als 60 Gew.-%, bezogen auf alle Acylreste R¹ beträgt,
   mit R² Methyl,
   wobei a = 1,8-2,2 und b = 1,8-2,2, mit der Maßgabe, dass a + b = 4.
In diesem Zusammenhang ist es insbesondere bevorzugt, wenn der Massenanteil an Resten R¹ von ungesättigten, linearen Fettsäuren von 18 Kohlenstoffatomen von 15 Gew.-% bis 30 Gew.-% bezogen auf alle Acylreste R¹ beträgt.

Bevorzugte Konsistenzgeber, die in den erfindungsgemäße Emulsionen bevorzugt enthalten sind, sind ausgewählt aus der Gruppe bestehend aus
Fettalkohole mit 12 bis 24 Kohlenstoffatomen, bevorzugt 14 bis 22 Kohlenstoffatomen, besonders bevorzugt 16 - 18 Kohlenstoffatomen,
Glycerinester und Polyglycerylestern,
Fettsäuren mit 12 bis 24 Kohlenstoffatomen und
polymerbasierte Verdicker.
Bei den Konsistenzgebern handelt es sich dabei beispielsweise um:
F ettalkohole mit 12 bis 24 C-Atomen, bevorzugt 14 bis 22, besonders bevorzugt 16 - 18,
Glycerylester oder Polyglycerylester, bevorzugt mit einem mittleren Polymerisationsgrad von maximal 4, mit mindestens einer Alkylkette mit 12 bis 24 C-Atomen, bevorzugt 14 bis 22, besonders bevorzugt 16 - 18,
Fettsäuren mit 12 bis 24 C-Atomen, bevorzugt 14 bis 22, besonders bevorzugt 16 - 18, Polymerbasierte Verdicker wie z.B. Cellulose, Cellulosederivate wie z.B.
Hydroxyethylcellulose, Hydroxypropylcellulose, alkylmodifizierte Cellulosetypen, wie beispielsweise Cetyl Hydroxyethylcellulose, Guar, alkylmodifizierte Guartypen, wie beispielsweise C18-22 Hydroxylalkyl Hydroxypropyl Guar, Stärkederivate, alkylmodifizierte Stärkederivate, Xanthan Gum, Gellan Gum, sowie Polyquaternium-Verbindungen wie beispielsweise Polyquaternium-37.

Als kosmetische Öle können Substanzen wie Silikonöle, funktionalisierte Silikone, Mineralöle, Fettsäureester, Fettalkoholether, natürliche Öle wie pflanzliche Öle, tierische Öle sowie deren Mischungen eingesetzt werden.
Bevorzugte Öle, die in den erfindungsgemäße Emulsionen bevorzugt enthalten sind, sind ausgewählt aus der Gruppe bestehend aus
Silikonöle, funktionalisierte Silikone, Mineralöle, Fettsäureester, Fettalkoholether, natürliche Öle wie pflanzliche Öle, tierische Öle Bevorzugte erfindungsgemäße Emulsionen enthalten als kosmetisches Öl mindestens eines ausgewählt aus der Gruppe umfassend, bevorzugt bestehend aus, Cyclopentasiloxane, Cyclomethicone, Dimethicone, Dimethiconol, Amodimethicone, PEG/PPG Dimethicones, Cetyl Dimethicone, Stearyl Dimethicone, Stearoxy Dimethicone, Behenoxy Dimethicone, Polyisobutene, Petrolatum, Mineral Oil, Hydrogenated Polydodecene, Hydrogenated Polydecene, Polydecene, Isoamyl Cocoate, PPG-3 Myristyl Ether, PPG-11 Stearyl Ether, Dicaprylyl Ether, Dicaprylyl Carbonate, Cetearyl Isononanoate, Cetyl Ethylhexanoate, Diethyhexyl Carbonate, Cetyl Ricinoleate, Myristyl Myristate, Stearyl Heptanoate, Decyl Cocoate, Decyl Oleate, PPG-15 Stearyl Ether, Octyldodecanol, Isocetyl Palmitate, Cetearyl Ethylhexanoate, Ethylhexyl Palmitate, Ethylhexyl Stearate, Isopropyl Palmitate, PPG-14 Butyl Ether, Triisostearin, C12-15 Alkyl Benzoate, Phenoxyethyl Caprylate, Isopropyl Myristate, Isoamyl Cocoate, Caprylic/Capric Triglyceride, Sonnenblumenöl, Olivenöl, Arganöl, Mineralöl, Castoröl, Ricinusöl, Kakaoöl, Palmöl, Cocosöl, Avocadoöl, Mandelöl, Jojobaöl, Sojaöl, Maisöl, Rapsöl, Sesamöl, Sojaöl, Weizenkeimöl, Walnußöl und Oleyl Erucate.

Bei den erfindungsgemäßen Emulsionen handelt es sich vorzugsweise um Öl-in-Wasser (O/W) Emulsionen.
Daher enthalten die erfindungsgemäßen Emulsionen bevorzugt Wasser, insbesondere in einer Menge von 20 Gew.-% bis 90 Gew.-%, bevorzugt 30 Gew.-% bis 85 Gew.-%, besonders bevorzugt 50 Gew.-% bis 80 Gew.-%, wobei sich die Gewichtsprozente auf die Gesamtemulsion beziehen.

Die erfindungsgemäßen Emulsionen sind insbesondere kosmetische Pflege- und Reinigungsformulierungen, insbesondere für Haut und Hautanhangsgebilde. Auch ist der Einsatz der erfindungsgemäßen Emulsionen in Sonnenschutz, Make-up und AP/Deo Produkten vorteilhaft.

Unter dem Begriff "Pflegeformulierung" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern.

Erfindungsgemäße Emulsionen umfassen somit insbesondere Cremes, Lotionen und Sprays zur Körper-, Gesichts-, Hand- und Fußpflege, Sonnenschutzemulsionen, Make-up Emulsionen, Mascaraprodukte, AP/Deo Emulsionen, Seren, Tränkemulsionen für Feuchttücher, Make-up Remover, BB (blemish balm) oder CC (color control) Cremes. Auch ist die Verwendung der erfindungsgemäßen Emulsionen als Leave-in Conditioner, Haarkur oder in ölhaltigen Shampoos und Duschbädern möglich.

Erfindungsgemäße kosmetische Pflege- und Reinigungsformulierungen, können zum Beispiel mindestens eine zusätzliche Komponente enthalten, ausgewählt aus der Gruppe der
nichtionischen Tenside,
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.
Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.

Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Ein weiterer Gegenstand der vorliegenden Erfindung ist die Verwendung der in den erfindungsgemäßen Formulierungen enthaltenen Alkylesterquats der allgemeinen Formel I als Emulgator, bevorzugt ÖI-in-Wasser-Emulgator, insbesondere in kosmetischen Pflege- und Reinigungsformulierungen, insbesondere für Haut und Hautanhangsgebilde.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: MDIPA Dialkylquat mit im Wesentlichen Stearinsäure

932 g (3,3 mol) Stearinfettsäure (98 % rein) werden mit 253 g (1,72 mol) Methyldiisopropanolamin gemischt und unter Rühren auf 180 °C aufgeheizt.

Reaktionswasser wird kontinuierlich abdestilliert. Nachdem die Hauptmenge Reaktionswassser bei Normaldruck destilliert ist, wird Vakuum angelegt und die Säurezahl der Reaktionsmischung auf < 7 mg KOH/g abreagiert. Das entstandene Esteramin wird auf 60 °C abgekühlt und portionsweise mit 197 g (1,56 mol) Dimethylsulfat versetzt, so dass die Reaktionstemperatur 100 °C nicht übersteigt. Nach dem Abkühlen auf Raumtemperatur wird die Gesamt Amin Zahl (GAZ) und der Aktivgehalt des Fertigprodukts analysiert.
GAZ = 4,5 mg KOH/g; Aktivgehalt 1,22 meq/g

### Beispiel 2: MDIPA Dialkylquat mit C16-18 Fettsäure mit ungesättigten Anteilen (< 30% ungesättigter Anteil)

1075 g (3,9 mol) Fettsäure C 16-18 und C 18 ungesättigt (lodzahl 20,2) werden mit 297 g (2,02 mol) Methyldiisopropanolamin gemischt und analog Beispiel 1 verestert.
Die Säurezahl des Esteramins betrug 6,5 mg KOH/g. Diese Mischung wurde wie im Beispiel 1 beschrieben mit 229,5 g (1,82 mol) DMS (Dimethylsulfat)umgesetzt. Das Fertigprodukt hatte eine GAZ von 4,9 mg KOH/g und einen Aktivgehalt von 1,29 meq/g.

### Beispiel 3: Herstellung von"1-Propanaminium, 2-hydroxy-N-(2-hydroxypropyl)-N,N-dimethyl-, diester mit Palmfettsäure, Methylsulfat"

1020 g (4 mol) Palmitinsäure (technische Qualität, ca. 98 % rein) werden mit 302 g (2,05 mol) Methyldiisopropanolamin versetzt und wie unter Beispiel 1 beschrieben verestert. Das Esteramin hatte eine Säurezahl von 5,6 mg KOH/g. Diese Mischung wurde wie unter Beispiel 1 beschrieben mit 240 g (1,90 mol) Dimethylsulfat alkyliert. Die GAZ des Fertigprodukts wurde mit 4,8 mg KOH/g bestimmt, der Aktivgehalt war 1,33 meq/g.

### Beispiel 4: MDIPA Dialkylquat mit C22 Fettsäure gesättigt

684,6 g (2,0 mol) Behensäure (Edenor C 22 85 GW) werden mit 152,5 g (1,03 mol) Methyldiisopropanolamin wie in Beispiel 1 beschrieben verestert. Das Esteramin hatte eine Säurezahl von 6,1 mg KOH/g. Die Reaktionsmischung wird mit 58,4 g (0,464 mol) DMS versetzt und im Temperaturbereich von 80-100 °C für 30 Minuten gerührt.

Anschließend gibt man 100 ml Isopropanol hinzu und dosiert weitere 58,4 g (0,464 mol) DMS so hinzu, dass die Temperatur im Bereich 80-100 °C gehalten werden kann. Das Produkt hatte eine GAZ von 5,5 mg KOH/g und einen Aktivgehalt von 0,97 meq/g.

### Beispiel 5: MDIPA Dialkylquat mit überwiegend C12-18 Fettsäure gesättigt

887 g (4,0 mol) Kokosfettsäure C 12-18 (Edenor K 1218) werden mit 305 g (2,06 mol) Methyldiisopropanolamin gemischt und wie unter Beispiel 1 beschrieben verestert. Die Reaktionsmischung hatte eine Säurezahl von 5,9 mg KOH/g. Das Reaktionsgemisch wird anschließend mit 233,3 g (1,85 mol) DMS analog Beispiel 1 alkyliert. Das Fertigprodukt hatte eine GAZ von 5,2 mg KOH/g und einen Aktivgehalt von 1,49 meq/g.

### Beispiel 6: MDIPA Dialkylquat mit Palmstearin Fettsäure

1378 g (5,17 mol) Palmstearin Fettsäure (lodzahl 34,2) werden mit 385,5 g (2,62 mol) Methldiisopropanolamin versetzt und wie unter Beispiel 1 beschrieben zur Reaktion gebracht. Das Esteramin hatte eine Säurezahl von 6,7 mg KOH/g. Diese Reaktionsmischung wurde analog Beispiel 1 mit 300,6 g (2,38 mol) DMS alkyliert. Das Fertigprodukt hatte eine GAZ von 5,1 mg KOH/g und einen Aktivgehalt von 1,32 meq/g.

### Vergleichsbeispiel 1: Herstellung von "1-Propanaminium, 2-hydroxy-N-(2-hydroxypropyl)-N,N-dimethyl-, diester mit Pflanzenmischölfettsäure, Methylsulfat"

Entsprechend Beispiel 1 der EP2783677 wurden 1120 g (4 mol) Pflanzenmischölfettsäure werden mit 302 g (2,05 mol) Methyldiisopropanolamin gemischt unter Rühren auf 180 °C aufgeheizt. Reaktionswasser wird kontinuierlich abdestilliert. Nachdem die Hauptmenge Reaktionswassser bei Normaldruck destilliert ist, wird Vakuum angelegt und die Säurezahl der Reaktionsmischung auf < 7 mg KOH/g abreagiert. Das entstandene Esteramin wird auf 60 °C abgekühlt und portionsweise mit 240 g (1,90 mol) Dimethylsulfat versetzt, so dass die Reaktionstemperatur 100 °C nicht übersteigt.

Nach dem Abkühlen auf Raumtemperatur wird die Gesamt Amin Zahl (GAZ) und er Aktivgehalt des Fertigprodukts analysiert.
GAZ = 5,0 mg KOH/g; Aktivgehalt 1,27meq/g (Kationischer Aktivgehalt nach Epton).

### Vergleichsbeispiel 2: nach INCI Distearoylethyl Dimonium Chloride; Cetearyl Alcohol

Hier wurde das kommerzielle Produkt VARISOFT EQ 65 von Evonik Industries AG eingesetzt.

### Vergleichsbeispiel 3: nach INCI Dipalmoylethyl Hydroxyethyl Methylammonium Methosulfate; Isopropanol

Hier wurde das kommerzielle Produkt REWOQUAT WE 28 von Evonik Industries AG eingesetzt.

### Lagerstabilität

In den Formulierungsbeispielen 1-1 bis 1-6 wird im Vergleich zu den Beispielen V1 bis V4 gezeigt, dass mit Hilfe erfindungsgemäßer Emulgatoren stabile O/W Emulsionen hergestellt werden können. Die Emulsionen wurden jeweils in einem klassischen Heiß-Heiß Prozess hergestellt, bei dem ÖI- und Wasserphase getrennt auf ca. 75°C erhitzt wurden. Anschließend wurde die Ölphase unter Rühren zur Wasserphase gegeben und anschließend für 60 Sekunden homogenisiert. Abschließend wurde bei ca. 30°C das Konservierungsmittel zugegeben, kurz gerührt und anschließend der pH-Wert auf ca. 6 eingestellt.

Die erfindungsgemäßen Emulsionsbeispiele waren dabei für mindestens drei Monate bei Raumtemperatur, 5°C, 40°C und 45°C stabil und überstanden erfolgreich auch einen dreimaligen Gefrier-Tau-Zyklus zwischen Raumtemperatur und -15°C.

Die Emulsionen mit den nichterfindungsgemäßen Vergleichsemulsionen zeigten dagegen insbesondere bei Wärmelagerung schnell Wasserabscheidung und somit keine für kosmetische Formulierungen akzeptable Stabilitäten.

Die gewählten Emulsionsrezepturen entsprechen dabei typischen Emulsionssystemen, wie sie beispielsweise auch auf Basis des Emulgators Distearyldimonium Chloride verwendet werden.

**Tabelle 1a: Vergleichsversuche zur Emulsionsstabilität**

| **Formulierungsbeispiel** | **1-1** | **1-2** | **1-3** | **1-4** | **1-5** |
|---|---|---|---|---|---|
| Beispiel 1 | 3,75% | | | | |
| Beispiel 2 | | 3,75% | | | |
| Beispiel 3 | | | 3,75% | | |
| Beispiel 4 | | | | 3,75% | |
| Beispiel 5 | | | | | 3,75% |
| Glyceryl Stearate | 1.0% | 1.0% | 1.0% | 1.0% | 1.0% |
| Cetearyl Alcohol | 1.25% | 1.25% | 1.25% | 1.25% | 1.25% |
| Mineral Oil | 4.0% | 4.0% | 4.0% | 4.0% | 4.0% |
| Isopropyl Palmitate | 4.0% | 4.0% | 4.0% | 4.0% | 4.0% |
| Caprylic/Capric Triglyceride | 4.0% | 4.0% | 4.0% | 4.0% | 4.0% |
| Dimethicone | 1.0% | 1.0% | 1.0% | 1.0% | 1.0% |
| Glycerin | 3,0% | 3,0% | 3,0% | 3,0% | 3,0% |
| Demineralized Water | ad 100% | ad 100% | ad 100% | ad 100% | ad 100% |
| Phenoxyethanol; Ethylhexylg lycerin¹⁾ | 0.7% | 0.7% | 0.7% | 0.7% | 0.7% |
| Sodium Hydroxide (10% aq., pH-Wert-Einstellung auf ca. 6) | q.s. | q.s. | q.s. | q.s. | q.s. |
| | | | | | |
| Stabilität | Stabil | Stabil | Stabil | Stabil | Stabil |

| | | | | | |
|---|---|---|---|---|---|
| ¹⁾ Euxyl PE 9010 (Schülke) | | | | | |

**Tabelle 1b: Vergleichsversuche zur Emulsionsstabilität**

| **Formulierungsbeispiel** | **1-6** | **V1** | **V2** | **V3** | **V4** |
|---|---|---|---|---|---|
| Beispiel 6 | 3,75% | | | | |
| Vergleichsbeispiel 1 | | 3,75% | | | |
| Vergleichsbeispiel 2 | | | 3,75% | | 5,75 |
| Vergleichsbeispiel 3 | | | | 3,75% | |
| Glyceryl Stearate | 1.0% | 1.0% | 1.0% | 1.0% | 0,25 |
| Cetearyl Alcohol | 1.25% | 1.25% | 1.25% | 1.25% | |
| Mineral Oil | 4.0% | 4.0% | 4.0% | 4.0% | 4.0% |
| Isopropyl Palmitate | 4.0% | 4.0% | 4.0% | 4.0% | 4.0% |
| Caprylic/Capric Triglyceride | 4.0% | 4.0% | 4.0% | 4.0% | 4.0% |
| Dimethicone | 1.0% | 1.0% | 1.0% | 1.0% | 1.0% |
| Glycerin | 3,0% | 3,0% | 3,0% | 3,0% | 3,0% |
| Demineralized Water | ad 100% | ad 100% | ad 100% | ad 100% | ad 100% |
| Phenoxyethanol; Ethylhexylg lycerin¹⁾ | 0.7% | 0.7% | 0.7% | 0.7% | 0.7% |
| Sodium Hydroxide (10% aq., pH-Wert-Einstellung auf ca. 6) | q.s. | q.s. | q.s. | q.s. | q.s. |
| | | | | | |
| Stabilität | Stabil | Wasserseparation nach 1 Woche 45°C | Wasserseparation nach 4 Wochen 45°C | Wasserseparation nach 4 Wochen 45°C | Wasserseparation nach 6 Wochen 45°C |

| | | | | | |
|---|---|---|---|---|---|
| ¹⁾ Euxyl PE 9010 (Schülke) | | | | | |

Dabei zeichnen sich die erfindungsgemäßen Emulsionen 1 bis 6 auch durch das typische Hautgefühl aus, wie man es beispielsweise mit vergleichbaren Emulsionen auf Basis des Emulgators Distearyldimonium Chloride erhalten kann. Die Emulsionen haben eine trockene, nichtfettige Sensorik und zeigen sich durch schnelle Absorption in die Haut und ein trockenes Hautgefühl nach dem Einziehen in die Haut aus. Weiterhin galt es zu demonstrieren, dass die erfindungsgemäßen Emulgatoren eine deutlich verbesserte Bioabbaubarkeit und eine deutlich verbesserte Ecotoxizität zeigen als der bisher am Markt übliche Standard des Dialkylquats Distearyldimonium Chloride. Daher wurde ein erfindungsgemäßer Emulgator (Beispiel 2) im Vergleich zu Distearyldimonium Chloride mit Hilfe verschiedener OECD Tests untersucht. Die Ergebnisse zeigen eine deutlich bessere Bioabbaubarkeit des erfindungsgemäßen Emulgators. Weiterhin findet man für die Langzeittoxizität gegenüber Fischen und für die Kurz- und Langzeittoxizität gegenüber wirbellosen Wassertieren deutlich erhöhte und daher vorteilhafte Grenzwerte für den erfindungsgemäßen Emulgator aus Beispiel 2.

**Tabelle 2: Vergleich der Bioabbaubarkeit und ausgewählter Ecotoxizitätswerte**

| | Distearyldimonium Chloride* | Beispiel 2 |
|---|---|---|
| Bioabbaubarkeit in Wasser | OECD 301: 3% (28d) | OECD 301: 60% (28d) |
| Langzeittoxizität gegenüber Fischen | Fish Early Life Stage Test (OECD 210) (Fathead minnow) NOEC=0.23 mg/l in standard water | Fish Early Life Stage Test (OECD 210) (Fathead minnow) NOEC=0.686 mg/l in standard water |
| Kurzzeittoxizität gegenüber wirbellosen Wassertieren | Acute Immobilisation Test (OECD 202) EC50(48h)=0.16 mg/l | Acute Immobilisation Test (OECD 202) EC50(48h)=6.7 mg/l |
| Langzeittoxizität gegenüber wirbellosen Wassertieren | Daphnia Magna Reproduction Test (OECD 211) NOEC(21d)=0.38 mg/l | Daphnia Magna Reproduction Test (OECD 211) NOEC(21d)=1 mg/l |

| | | |
|---|---|---|
| * VARISOFT TA 100 (Evonik Industries AG) | | |

Die Beispiele belegen somit eindeutig, dass die erfindungsgemäßen Emulsionssysteme deutliche Vorteile gegenüber dem bekannten Stand der Technik bieten, indem sie zeigen, wie umweltfreundliche kationische Emulsionssysteme für kosmetische Pflegeanwendungen darstellbar sind.

### Weitere Formulierungsbeispiele

Die Konzentrationsangaben in den folgenden Rezepturen sind in Massenprozent.

### O/W Lotion

Dieses Beispiel soll zeigen, dass es auch sehr gut möglich ist, den erfindungsgemäßen Emulgator der Wasserphase zuzusetzen.

| | |
|---|---|
| Beispiel 1 | 5.0 % |
| Water | ad 100 % |
| Glycerin | 3.0 % |
| Glyceryl Stearate²⁾ | 1.0 % |
| Cetearyl Alcohol | 1.25 % |
| Stearyl Alcohol | 1.0 % |
| Mineral Oil | 3.6 % |
| Isopropyl Palmitate | 3.6 % |
| Caprylic/Capric Triglyceride | 3.6 % |
| Dimethicone | 1.0 % |
| Phenoxyethanol; Ethylhexylglycerin¹⁾ | 0,7 % |

| | |
|---|---|
| ¹⁾ Euxyl PE 9010 (Schülke) ²⁾ TEGIN M (Evonik Industries AG) | |

### Sonnenschutzemulsion mit SPF 50

| | |
|---|---|
| Beispiel 1 | 3.0 % |
| Glyceryl Stearate | 2,0 % |
| Stearyl Alcohol | 1,5% |
| Stearic Acid | 2,0 % |
| C12-15 Alkyl Benzoate | 5,0 % |
| Phenoxyethyl Caprylate | 2,0 % |
| Diethylhexyl Carbonate | 3,0 % |
| Dimethicone | 1,0 % |
| Butyl Methoxydibenzoylmethane | 5,0 % |
| Octocrylene | 10,0 % |
| Triacontanyl PVP³⁾ | 2,0 % |
| Titanium Dioxide; Silica; Glycerin⁴⁾ | 5,5 % |
| Titanium Dioxide; Silica⁵⁾ | 7,5 % |
| Demineralized Water | ad 100 % |
| Glycerin | 3,0 % |
| EDTA | 0,1 % |
| Cetearyl Glucoside⁶⁾ | 2,0 % |
| Polyquaternium-37⁷⁾ | 1,0 % |
| Phenoxyethanol; Ethylhexylglycerin¹⁾ | 0,7 % |

| | |
|---|---|
| ³⁾ Ganex WP-660 (Ashland Specialty Chemicals) ⁴⁾ UV Titan M040 (Merck Performane Materials) ⁵⁾ Eusolx T-Avo (Merck Performance Materials) ⁶⁾ TEGO Care CG 90 (Evonik Industries AG) ⁷⁾ Cosmedia Ultragel 300 (BASF Personal Care and Nutrition GmbH) | |

### AP/Deo Lotion

| | |
|---|---|
| Beispiel 2 | 1,0% |
| Steareth-2 | 3.0% |
| Steareth-20 | 2.0% |
| Diethylhexyl Carbonate | 3,0% |
| PPG-14 Butyl Ether | 3,0% |
| Polyglyceryl-3 Caprylate | 0,5% |
| Demineralized Water | ad 100% |
| Hydroxyethylcellulose ⁸⁾ | 1,0% |
| Aluminum Chlorohydrate (50%ig) ⁹⁾ | 15,0% |
| Phenoxyethanol; Ethylhexylglycerin¹⁾ | 0,8% |

| | |
|---|---|
| ⁸⁾ Natrosol 250 HHR (Ashland Specialty Chemicals) ⁹⁾ Reach 501L (Reheis) | |

### Lotion mit kosmetischen Wirkstoffen

| | |
|---|---|
| Beispiel 2 | 2.0% |
| Polyglyceryl-6 Stearate; Polyglyceryl-6 Behenate¹⁰ | 1.5 |
| Glyceryl Stearate | 0,5% |
| Cetearyl Alcohol | 0,5% |
| Caprylic/Capric Triglyceride | 8,5% |
| Ethylhexyl Palmitate | 8,5% |
| Demineralized Water | ad 100% |
| Polyquaternium-37⁷⁾ | 0,8% |
| Tetrapeptide-21; Glycerin; Butylene Glycol; Aqua ¹¹⁾ | 0.5% |
| Creatine¹²⁾ | 0.2% |
| Caffeine | 0.2% |
| Phenoxyethanol, Methylparaben, Propylparaben; Ethylparaben¹³⁾ | 1.0 % |

| | |
|---|---|
| ¹⁰⁾ TEGO Care PBS 6 (Evonik Industries AG) ¹¹⁾TEGO Pep 4-17 (Evonik Industries AG) ¹²⁾TEGO Cosmo C 100 (Evonik Industries AG) ¹³⁾ Phenonip XB (Clariant International Ltd.) | |

### O/W Serum

| | |
|---|---|
| Beispiel 2 | 3,0% |
| Cetearyl Glucoside⁶⁾ | 0,5% |
| Glyceryl Stearate | 0,75% |
| Stearyl Alcohol | 0,75% |
| Caprylic/Capric Triglyceride | 2,0% |
| Oleyl Erucate | 2,0% |
| Isoamyl Cocoate | 3,0% |
| Persea Gratissima (Avocado) Oil | 1,0% |
| Aqua; Ethylhexyl Stearate; Sodium Hyaluronate Crosspolymer; Polyglyceryl-4 Diisostearate/Polyhydroxystearate/Sebacate; Sodium Isostearate ¹⁴⁾ | |
| Demineralized Water | ad 100% |
| Butylene Glycol | 5,0% |
| Tetrapeptide-21; Glycerin; Butylene Glycol; Aqua ¹¹⁾ | 2,0% |
| Hydrolyzed Hyaluronic Acid ¹⁵⁾ | 0,1% |
| Polyquaternium-37⁷⁾ | 0,5% |
| Phenoxyethanol; Ethylhexylglycerin¹⁾ | 0,8% |

| | |
|---|---|
| ¹¹⁾ HYACARE Filler CL (Evonik Industries AG) ¹¹⁾ HYACARE 50 (Evonik Industries AG) | |

### Sprühbare Lotion

| | |
|---|---|
| Beispiel 2 | 4,5% |
| Glycerin | 4.0% |
| Demineralized Water | ad 100% |
| Diethylhexyl Carbonate | 2.5% |
| Isopropyl Palmitate | 4.25% |
| Ceteareth-25 | 1.5% |
| Stearyl Alcohol | 4.0% |
| Cetyl Dimethicone¹⁶⁾ | 2.5% |
| Mineral Oil | 1.0% |
| Phenoxyethanol; Ethylhexylglycerin¹⁾ | 0,7% |

| | |
|---|---|
| ¹⁶⁾ ABIL Wax 9801 (Evonik Industries AG) | |

### Kationische Sonnencreme

| | |
|---|---|
| Beispiel 1 | 5.0% |
| Glyceryl Stearate | 1.0% |
| Stearyl Alcohol | 1.0% |
| Cetearyl Alcohol | 1.0% |
| C12-15 Alkyl Benzoate | 4.0% |
| Diethylhexyl Carbonate | 3.0% |
| Cetyl Ricinoleate | 1.0% |
| Triisostearine | 1.0% |
| Butyl Methoxydibenzoylmethane | 2.0% |
| Ethylhexyl Salicylate | 4.0% |
| Octocrylene | 7.0% |
| Creatine¹²⁾ | 0.5% |
| Glycerin | 3.0% |
| Demineralized Water | ad 100% |
| Phenoxyethanol; Ethylhexylglycerin¹⁾ | 0,7% |

### Kationische Foundation

| | |
|---|---|
| Beispiel 4 | 4.8% |
| Glyceryl Stearate | 1.0% |
| Stearyl Alcohol | 1.0% |
| Cetearyl Alcohol | 1.0% |
| Ethylhexyl Palmitate | 4.0% |
| Diethylhexyl Carbonate | 4.0% |
| Titanium Dioxide¹⁷⁾ | 8.0% |
| Iron Oxide¹⁸⁾ | 4.0% |
| Iron Oxide¹⁹⁾ | 1.8% |
| Iron Oxide²⁰⁾ | 0.4% |
| Glycerin | 3.0% |
| Demineralized Water | ad 100% |
| Phenoxyethanol; Ethylhexylglycerin¹⁾ | 0,7% |

| | |
|---|---|
| ¹⁷⁾ Hombitan AC 360 (Sachtleben) ¹⁸⁾ Unipure Yellow LC 182 (Sensient Technologies) ¹⁹⁾ Unipure Red LC 381 (Sensient Technologies) ²⁰⁾ Unipure Black LC 989 (Sensient Technologies) | |

### O/W Softcreme

| | |
|---|---|
| Beispiel 3 | 2.0% |
| Bis-PEG/PPG-16/16 PEG/PPG-16/16 Dimethicone; Caprylic/Capric Triglyceride²¹⁾ | 1.0% |
| Glyceryl Stearate | 2.5% |
| Cetearyl Alcohol | 1.5% |
| Cetearyl Ethylhexanoate | 9.0% |
| Caprylic/Capric Triglyceride | 9.0% |
| Glycerin | 8.0% |
| Demineralized Water | ad 100% |
| Phenoxyethanol, Methylparaben, Propylparaben; Ethylparaben¹³⁾ | 0,7% |

| | |
|---|---|
| ²¹⁾ ABIL Care 85 (Evonik Industries AG) | |

### O/W Blemish Balm Lotion

| | |
|---|---|
| Beispiel 1 | 2,0% |
| Polyglyceryl-6 Stearate; Polyglyceryl-6 Behenate¹⁰⁾ | 2.0% |
| Glyceryl Stearate | 0,75% |
| Stearyl Alcohol | 0,75% |
| Diethylhexyl Carbonate | 7,40% |
| Ethylhexyl Methoxycinnamate | 5,00% |
| Diethylamino Hydroxybenzoyl Hexyl Benzoate ²²⁾ | 3,00% |
| Phytosphingosine | 0,10% |
| Hydrolyzed Hyaluronic Acid ¹⁵⁾ | 0,10% |
| Tetrapeptide-21; Glycerin; Butylene Glycol; Aqua ¹¹⁾ | 2,00% |
| Demineralized Water | ad 100% |
| Titanium Dioxide ¹⁷⁾ | 3,00% |
| Talc | 2,00% |
| Iron Oxide ¹⁸⁾ | 0,36% |
| Iron Oxide ¹⁹⁾ | 0,12% |
| Iron Oxide ²⁰⁾ | 0,08% |
| Isoamyl Cocoate | 4,44% |
| Phenoxyethyl Caprylate | 4,00% |
| Nylon-10/10 ²³⁾ | 3,00% |
| Glycerin | 3,00% |
| Polyquaternium-37⁷⁾ | 0,50% |
| Ethanol | 3,00% |
| Methylisothiazolinone, Methylparaben, Ethylparaben; Dipropylene Glycol ²⁴⁾ | 0,80% |

| | |
|---|---|
| ²²⁾ Uvinul A Plus (BASF SE) ²³⁾ TEGOLON ECO 10-10 (Evonik Industries AG) ²⁴⁾ Microcare MEM (Thor Personal Care) | |

### Konservierungsmittelfreie Lotionen

| | | |
|---|---|---|
| Beispiel 2 | 3,0% | 3,0% |
| Glyceryl Stearate | 0,5% | 0,5% |
| Stearyl Alcohol | 0,5% | 0,5% |
| Prunus Amygdalus Dulcis (Sweet Almond) Oil | 10,0% | 10,0% |
| Isoamyl Cocoate | 6,6% | 6,0% |
| Glycerin | 4,0% | 4,0% |
| Demineralized Water | ad 100,0% | ad 100,0% |
| Caprylyl Glycol, Glycerin, Glyceryl Caprylate, Phenylpropanol ²⁵⁾ | 1,0% | |
| Methylpropanediol, Caprylyl Glycol, Phenylpropanol ²⁶⁾ | | 4,0% |
| Polyquaternium-37⁷⁾ | 0,5% | 0,5% |

| | | |
|---|---|---|
| ²⁵⁾ Dermosoft LP (Dr. Straetmans Chemische Produkte GmbH) ²⁶⁾ Dermosoft OMP (Dr. Straetmans Chemische Produkte GmbH) | | |

### O/W Softcreme

| | |
|---|---|
| Beispiel 2 | 2.0% |
| Bis-PEG/PPG-16/16 PEG/PPG-16/16 Dimethicone; Caprylic/Capric Triglyceride²¹⁾ | 1.0% |
| Glyceryl Stearate | 2.5% |
| Cetearyl Alcohol | 1.5% |
| Diethylhexyl Carbonate | 5.0% |
| Dimethicone | 5.0% |
| Dimethicone/Vinyl Dimethicone Crosspolymer ²⁷⁾ | 0.5% |
| Cyclopentasiloxane; Dimethicone Crosspolymer ²⁸⁾ | 5.0% |
| Glycerin | 3.0% |
| Demineralized Water | ad 100% |
| Methylisothiazolinone, Methylparaben, Ethylparaben; Dipropylene Glycol ²⁴⁾ | 0,8% |

| | |
|---|---|
| ¹¹⁾ KSG-15 (Shin Etsu) ²⁸⁾ DC 9045 Elastomer Blend (Dow Corning) | |

## Patentansprüche

1. Emulsion enthaltend
A) Alkylesterquats der allgemeinen Formel I 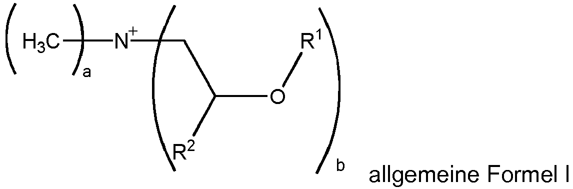
mit R¹ ein Acylrest einer Fettsäure mit einer Kettenlänge von 6 bis 24 Kohlenstoffatomen, bevorzugt 12 bis 22 Kohlenstoffatomen, insbesondere 16 bis 18 Kohlenstoffatomen, mit der Maßgabe, dass der Massenanteil von gesättigten, linearen Fettsäuren mit einer Kettenlänge von 12 bis 24 Kohlenstoffatomen, bevorzugt 14 bis 22 Kohlenstoffatomen, insbesondere 16 bis 18 Kohlenstoffatomen, mehr als 50 Gew.-%, bevorzugt mehr als 55 Gew.-%, ganz besonders bevorzugt mehr als 60 Gew.-%, bezogen auf alle Acylreste R¹ beträgt,
mit R² ein Alkylrest mit 1 bis 6 Kohlenstoffatomen, bevorzugt Methyl, Ethyl, Propyl, Isopropyl, besonders bevorzugt Methyl,
wobei a = 1-3 und b = 1-3, bevorzugt a = 1,8-2,2 und b = 1,8-2,2, besonders bevorzugt a = b = 2, mit der Maßgabe, dass a + b = 4,
B) mindestens einen Konsistenzgeber, und
C) mindestens ein kosmetisches Öl.

2. Emulsion gemäß Anspruch 1, **dadurch gekennzeichnet, dass**
A) in einer Menge von 0,2 Gew.-% bis 10 Gew.-%, bevorzugt von 0,5 Gew.-% bis 7 Gew.-% insbesondere von 1 Gew.-% bis 6 Gew.-%,
B) in einer Menge von 0,5 Gew.-% bis 10 Gew.-%, bevorzugt von 2 Gew.-% bis 8 Gew.-% insbesondere von 3 Gew.-% bis 6 Gew.-%, und
C) in einer Menge von 5 Gew.-% bis 40 Gew.-%, bevorzugt von 6 Gew.-% bis 35 Gew.-% insbesondere von 8 Gew.-% bis 30 Gew.-%,
wobei sich die Gewichtsprozent auf die Gesamtemulsion beziehen.

3. Emulsion gemäß mindestens einem der vorherigen Ansprüche, **dadurch gekennzeichnet, dass**
A) Alkylesterquats der allgemeinen Formel I
mit R¹ ein Acylrest einer Fettsäure mit einer Kettenlänge von 6 bis 24, mit der Maßgabe, dass der Massenanteil von gesättigten, linearen Fettsäuren mit einer Kettenlänge von 16 bis 18 Kohlenstoffatomen, mehr als 60 Gew.-%, bezogen auf alle Acylreste R¹ beträgt,
mit R² Methyl,
wobei a = 1,8-2,2 und b = 1,8-2,2, mit der Maßgabe, dass a + b = 4.

4. Emulsion gemäß mindestens einem der vorherigen Ansprüche, **dadurch gekennzeichnet, dass**
A) Alkylesterquats der allgemeinen Formel I
mit R¹ ein Acylrest einer Fettsäure mit einer Kettenlänge von 6 bis 24, mit der Maßgabe, dass der Massenanteil von gesättigten, linearen Fettsäuren mit einer Kettenlänge von 18 Kohlenstoffatomen, mehr als 60 Gew.-%, bezogen auf alle Acylreste R¹ beträgt,
mit R² Methyl,
wobei a = 1,8-2,2 und b = 1,8-2,2, mit der Maßgabe, dass a + b = 4.

5. Emulsion gemäß mindestens einem der vorherigen Ansprüche, **dadurch gekennzeichnet, dass** der Massenanteil an Resten R¹ von ungesättigten, linearen Fettsäuren mit einer Kettenlänge von 12 bis 24 Kohlenstoffatomen, bevorzugt 14 bis 22 Kohlenstoffatomen, insbesondere 16 bis 18 Kohlenstoffatomen, von 5 Gew.-% bis 35 Gew.-%, bevorzugt von 10 Gew.-% bis 30 Gew.-%, ganz besonders bevorzugt von 15 Gew.-% bis 25 Gew.-%, bezogen auf alle Acylreste R¹ beträgt.

6. Emulsion gemäß mindestens einem der vorherigen Ansprüche, **dadurch gekennzeichnet, dass** der Konsistenzgeber ausgewählt ist aus der Gruppe bestehend aus
Fettalkohole mit 12 bis 24 Kohlenstoffatomen, bevorzugt 14 bis 22 Kohlenstoffatomen, besonders bevorzugt 16 - 18 Kohlenstoffatomen, Glycerinester und Polyglycerylestern,
Fettsäuren mit 12 bis 24 Kohlenstoffatomen und
polymerbasierte Verdicker.

7. Emulsion gemäß mindestens einem der vorherigen Ansprüche, **dadurch gekennzeichnet, dass** das kosmetische Öl ausgewählt ist aus der Gruppe bestehend aus
Silikonöle, funktionalisierte Silikone, Mineralöle, Fettsäureester, Fettalkoholether, natürliche Öle wie pflanzliche Öle, tierische Öle

8. Emulsion gemäß mindestens einem der vorherigen Ansprüche, **dadurch gekennzeichnet, dass** sie eine kosmetische Pflege- und Reinigungsformulierung, insbesondere für Haut und Hautanhangsgebilde, darstellt.

9. Verwendung von Alkylesterquats der allgemeinen Formel I wie in einem der vorherigen Ansprüche beschrieben als Emulgator.

## Claims

1. Emulsion comprising
A) alkyl ester quats of the general formula I 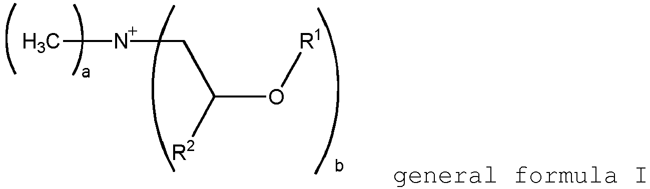
where R¹ is an acyl radical of a fatty acid with a chain length of from 6 to 24 carbon atoms, preferably 12 to 22 carbon atoms, in particular 16 to 18 carbon atoms, with the proviso that the mass fraction of saturated, linear fatty acids with a chain length of from 12 to 24 carbon atoms, preferably 14 to 22 carbon atoms, in particular 16 to 18 carbon atoms, is more than 50% by weight, preferably more than 55% by weight, very particularly preferably more than 60% by weight, based on all of the acyl radicals R¹,
where R² is an alkyl radical having 1 to 6 carbon atoms, preferably methyl, ethyl, propyl or isopropyl, particularly preferably methyl,
where a = 1-3 and b = 1-3, preferably a = 1.8-2.2 and b = 1.8-2.2, particularly preferably a = b = 2, with the proviso that a + b = 4,
B) at least one consistency regulator, and
C) at least one cosmetic oil.

2. Emulsion according to Claim 1, **characterized in that**
A) in an amount of from 0.2% by weight to 10% by weight, preferably from 0.5% by weight to 7% by weight, in particular from 1% by weight to 6% by weight,
B) in an amount of from 0.5% by weight to 10% by weight, preferably from 2% by weight to 8% by weight, in particular from 3% by weight to 6% by weight, and
C) in an amount of from 5% by weight to 40% by weight, preferably from 6% by weight to 35% by weight, in particular from 8% by weight to 30% by weight,
where the percent by weight refer to the total emulsion.

3. Emulsion according to at least one of the preceding claims, **characterized in that**
A) alkyl ester quats of the general formula I
where R¹ is an acyl radical of a fatty acid having a chain length of from 6 to 24, with the proviso that the mass fraction of saturated, linear fatty acids with a chain length of from 16 to 18 carbon atoms is more than 60% by weight, based on all of the acyl radicals R¹,
where R² is methyl,
where a = 1.8-2.2 and b = 1.8-2.2, with the proviso that a + b = 4.

4. Emulsion according to at least one of the preceding claims, **characterized in that**
A) alkyl ester quats of the general formula I
where R¹ is an acyl radical of a fatty acid having a chain length of from 6 to 24, with the proviso that the mass fraction of saturated, linear fatty acids with a chain length of 18 carbon atoms is more than 60% by weight, based on all of the acyl radicals R¹,
where R² is methyl,
where a = 1.8-2.2 and b = 1.8-2.2, with the proviso that a + b = 4.

5. Emulsion according to at least one of the preceding claims, **characterized in that** the mass fraction of radicals R¹ from unsaturated, linear fatty acids having a chain length of from 12 to 24 carbon atoms, preferably 14 to 22 carbon atoms, in particular 16 to 18 carbon atoms, is from 5% by weight to 35% by weight, preferably from 10% by weight to 30% by weight, very particularly preferably from 15% by weight to 25% by weight, based on all of the acyl radicals R¹.

6. Emulsion according to at least one of the preceding claims, **characterized in that** the consistency regulator is selected from the group consisting of fatty alcohols having 12 to 24 carbon atoms, preferably 14 to 22 carbon atoms, particularly preferably 16-18 carbon atoms,
glycerol esters and polyglyceryl esters,
fatty acids having 12 to 24 carbon atoms and polymer-based thickeners.

7. Emulsion according to at least one of the preceding claims, **characterized in that** the cosmetic oil is selected from the group consisting of silicone oils, functionalized silicones, mineral oils, fatty acid esters, fatty alcohol ethers, natural oils such as vegetable oils, animal oils.

8. Emulsion according to at least one of the preceding claims, **characterized in that** it is a cosmetic care and cleaning formulation, in particular for skin and skin appendages.

9. Use of alkyl ester quats of the general formula I as described in one of the preceding claims as emulsifier.

## Revendications

1. Émulsion contenant :
A) des alkylesterquats de formule générale I 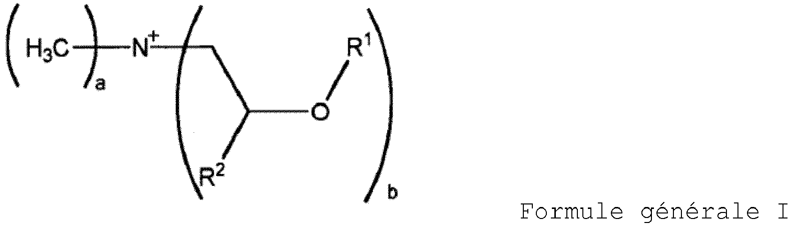
dans laquelle R¹ représente un radical acyle d'un acide gras ayant une longueur de chaîne de 6 à 24 atomes de carbone, de préférence de 12 à 22 atomes de carbone, notamment de 16 à 18 atomes de carbone, à condition que la proportion en masse d'acides gras linéaires saturés ayant une longueur de chaîne de 12 à 24 atomes de carbone, de préférence de 14 à 22 atomes de carbone, notamment de 16 à 18 atomes de carbone, soit de plus de 50 % en poids, de préférence de plus de 55 % en poids, de manière tout particulièrement préférée de plus de 60 % en poids, par rapport à tous les radicaux acyle R¹,
R² représente un radical alkyle de 1 à 6 atomes de carbone, de préférence méthyle, éthyle, propyle, isopropyle, de manière particulièrement préférée méthyle,
avec a = 1 à 3 et b = 1 à 3, de préférence a = 1,8 à 2,2 et b = 1,8 à 2,2, de manière particulièrement préférée avec a = b = 2, à condition que a + b = 4,
B) au moins un épaississant et
C) au moins une huile cosmétique.

2. Émulsion selon la revendication 1, **caractérisée en ce que**
A) en une quantité de 0,2 % en poids à 10 % en poids, de préférence de 0,5 % en poids à 7 % en poids, notamment de 1 % en poids à 6 % en poids,
B) en une quantité de 0,5 % en poids à 10 % en poids, de préférence de 2 % en poids à 8 % en poids, notamment de 3 % en poids à 6 % en poids,
C) en une quantité de 5 % en poids à 40 % en poids, de préférence de 6 % en poids à 35 % en poids, notamment de 8 % en poids à 30 % en poids,
les pourcentages en poids se rapportant à l'émulsion totale.

3. Émulsion selon au moins l'une quelconque des revendications précédentes, **caractérisée en ce que**
A) des alkylesterquats de formule générale I dans laquelle R¹ représente un radical acyle d'un acide gras ayant une longueur de chaîne de 6 à 24, à condition que la proportion en masse d'acides gras linéaires saturés ayant une longueur de chaîne de 16 à 18 atomes de carbone soit de plus de 60 % en poids, par rapport à tous les radicaux acyle R¹,
R² représente méthyle,
avec a = 1,8 à 2,2 et b = 1,8 à 2,2, à condition que a + b = 4.

4. Émulsion selon au moins l'une quelconque des revendications précédentes, **caractérisée en ce que**
A) des alkylesterquats de formule générale I
dans laquelle R¹ représente un radical acyle d'un acide gras ayant une longueur de chaîne de 6 à 24, à condition que la proportion en masse d'acides gras linéaires saturés ayant une longueur de chaîne de 18 atomes de carbone soit de plus de 60 % en poids, par rapport à tous les radicaux acyle R¹,
R² représente méthyle,
avec a = 1,8 à 2,2 et b = 1,8 à 2,2, à condition que a + b = 4.

5. Émulsion selon au moins l'une quelconque des revendications précédentes, **caractérisée en ce que** la proportion en masse de radicaux R¹ d'acides gras linéaires insaturés ayant une longueur de chaîne de 12 à 24 atomes de carbone, de préférence de 14 à 22 atomes de carbone, notamment de 16 à 18 atomes de carbone, est de 5 % en poids à 35 % en poids, de préférence de 10 % en poids à 30 % en poids, de manière tout particulièrement préférée de 15 % en poids à 25 % en poids, par rapport à tous les radicaux acyle R¹.

6. Émulsion selon au moins l'une quelconque des revendications précédentes, **caractérisée en ce que** l'épaississant est choisi dans le groupe constitué par : les alcools gras de 12 à 24 atomes de carbone, de préférence de 14 à 22 atomes de carbone, de manière particulièrement préférée de 16 à 18 atomes de carbone, les esters de glycérine et les esters de polyglycéryle, les acides gras de 12 à 24 atomes de carbone, et les épaississants à base de polymères.

7. Émulsion selon au moins l'une quelconque des revendications précédentes, **caractérisée en ce que** l'huile cosmétique est choisie dans le groupe constitué par :
les huiles de silicone, les silicones fonctionnalisées, les huiles minérales, les esters d'acides gras, les éthers d'alcools gras, les huiles naturelles telles que les huiles végétales, les huiles animales.

8. Émulsion selon au moins l'une quelconque des revendications précédentes, **caractérisée en ce qu'**il s'agit d'une formulation cosmétique de soin et de nettoyage, notamment pour la peau et les annexes de la peau.

9. Utilisation d'alkylesterquats de formule générale I tels que décrits dans l'une quelconque des revendications précédentes en tant qu'émulsifiants.
